# EUROPÄISCHE PATENTANMELDUNG

(11) **EP 2 260 761 A1**
(43) Veröffentlichungstag der Anmeldung: **15.12.2010**
(21) Anmeldenummer: 10182564.4
(22) Anmeldetag: 13.05.2005
(51) Int. Cl.: A61B 5/00

(54) **Zahnärztliche Vorrichtung zum Untersuchen der optischen Eigenschaften von Zahngewebe**

(30) Priorität: 14.05.2004 DE 102004024165
(62) Teilanmeldung aus: 05010492.6
(71) Anmelder: Kaltenbach & Voigt GmbH, 88400 Biberach (DE)
(72) Erfinder: Hack, Alexander, 88400, Biberach (DE); Hans, Heckenberg, 88433, Aßmannshardt (DE); Erdmann, Sven, 89081, Ulm (DE)
(74) Vertreter: Thun, Clemens

(57) **Zusammenfassung**

Bei einer zahnärztliche Vorrichtung zum Untersuchen der optischen Eigenschaften von Zahngewebe, insbesondere zum Erkennen von Karies, Plaque, bakteriellem Befall, Konkrementen und Zahnstein, mit Mitteln zum Erzeugen einer Anregungsstrahlung (A), welche auf einen zu untersuchenden Zahngewebebereich zu lenken ist, Erfassungsmitteln (40) und Auswertemitteln zum Erfassen und Bewerten einer von dem bestrahlten Zahngewebebereich als Antwort auf die Bestrahlung entstehenden Antwortstrahlung (F), sowie Anzeigemitteln (4b) zum Anzeigen eines von den Auswertemitteln auf Basis der erfassten Antwortstrahlung ermittelten Messergebnisses, sind die Mittel zum Erzeugen der Anregungsstrahlung (A), die Erfassungsmittel (40) sowie mindestens auch die Anzeigemittel (4b) in ein zahnärztliches Handstück (1) integriert.

## Beschreibung

Die vorliegende Erfindung betrifft eine zahnärztliche Vorrichtung zum Untersuchen der optischen Eigenschaften von Zahngewebe gemäß dem Oberbegriff des Anspruchs 1. Insbesondere betrifft die vorliegende Erfindung eine Vorrichtung zum Erkennen von fluoreszierenden Substanzen an Zähnen, beispielsweise von Karies, Plaque, bakteriellem Befall, Konkrementen oder Zahnstein.

In der Dentaldiagnostik sind optische Untersuchungsvorrichtungen, mit deren Hilfe beispielsweise Karies, Plaque, bakterieller Befall, Konkremente oder Zahnstein erkannt werden kann, bereits seit längerem und in unterschiedlichen Varianten bekannt. Allen bekannten Vorrichtungen ist gemeinsam, dass ein zu untersuchender Zahngewebebereich zunächst mit einer Anregungsstrahlung bestrahlt wird, woraufhin von dem Zahn eine Antwortstrahlung abgegeben wird. Diese Antwortstrahlung kann sowohl die zurückreflektierte Strahlung gleicher Wellenlänge als auch eine Fluoreszenzstrahlung beinhalten. Die Antwortstrahlung wird wiederum erfaßt und einer Auswerteeinheit zugeführt, welche anhand des Spektrums der Antwortstrahlung ermittelt, ob eine der oben genannten Substanzen vorhanden ist oder nicht. Die bekannten optischen Diagnoseverfahren und Vorrichtungen unterscheiden sich dabei durch die verwendete(n) Wellenlänge(n) für die Anregungsstrahlung sowie durch die Auswertung der erfaßten Antwortstrahlung. Eine erste Möglichkeit besteht darin, zu untersuchen, ob an dem Zahn eine Fluoreszenzstrahlung als Reaktion auf die Anregungsstrahlung hin entstanden ist. Eine andere Möglichkeit besteht bei der sogenannten Reflexionsspektrometrie darin, zu untersuchen, welche Wellenlängen von der Zahnoberfläche in welcher Weise reflektiert werden.

Vorrichtungen dieser Art sind beispielsweise in der DE 297 04 185 U1, der DE 197 09 500 C1 oder der DE 100 13 210 A1 beschrieben. Diese bekannten Vorrichtungen weisen ein zahnärztliches Handstück mit einer Untersuchungssonde auf, über welche die Anregungsstrahlung auf den zu untersuchenden Zahn gelenkt sowie die von dem Zahn zurückgestrahlte Antwortstrahlung erfaßt wird. Die Lichtquelle zur Erzeugung der Anregungsstrahlung ist dabei oftmals unmittelbar in dem Handstück angeordnet, während hingegen die Auswertung der Antwortstrahlung vorwiegend in einer Konsole erfolgt, welche über einen Verbindungsschlauch mit dem Handstück verbunden ist. Diese Konsole weist zum einen die Elektronik für die Ansteuerung der Lichtquelle für die Anregungsstrahlung als auch für die Auswertung der Antwortstrahlung auf. Darüber sind an der Konsole Anzeigemittel in Form eines Displays vorgesehen, welche das Meßergebnis darstellen und damit Auskunft darüber geben, ob der gerade untersuchte Bereich eine der oben genannten fluoreszierenden Substanzen aufweist oder nicht. Die abschließende Diagnose schließlich, d.h. die Feststellung, ob die erhöhte Fluoreszenz durch Karies hervorgerufen wird oder nicht, erstellt dann der Zahnarzt.

Der vorliegenden Erfindung liegt die Aufgabenstellung zugrunde, die Handhabung bislang bekannter zahnärztlicher optischer Untersuchungseinrichtungen weiter zu verbessern. Hierbei ist zu berücksichtigen, dass für einen Benutzer des Geräts die Möglichkeit bestehen sollte, auch schwer zugängliche Bereiche innerhalb des Mundraums eines Patienten untersuchen zu können. Ferner sollte die Möglichkeit einer einfachen Reinigung bestehen, da insbesondere diejenigen Teile, welche in den Mundraum des Patienten gelangen, auch sterilisiert werden müssen.

Die Aufgabenstellung wird durch die in den unabhängigen Ansprüchen definierte Erfindung gelöst.

Dabei wird gemäß einem ersten Aspekt der vorliegenden Erfindung vorgeschlagen, die zahnärztliche Untersuchungsvorrichtung derart auszugestalten, dass sie durch ein Handgerät gebildet ist, welches im Prinzip vollkommen unabhängig von weiteren Einrichtungen benutzt werden kann und alle wesentlichen und zur Durchführung des optischen Diagnoseverfahrens erforderlichen Elemente aufweist. Die erfindungsgemäße Vorrichtung besteht somit aus Mitteln zum Erzeugen einer Anregungsstrahlung, welche auf einen zu untersuchenden Zahngewebebereich zu lenken ist, Erfassungsmitteln und Auswertemitteln zum Erfassen und Bewerten einer von dem bestrahlten Zahngewebebereich als Antwort auf die Bestrahlung zurückgeworfenen Antwortstrahlung sowie Anzeigemitteln zum Anzeigen eines von den Auswertemitteln auf Basis der erfaßten Antwortstrahlung ermittelten Meßergebnisses, wobei die Mittel zum Erzeugen der Anregungsstrahlung, die Erfassungsmittel sowie zumindest auch die Anzeigemittel in bzw. an einem Handstück angeordnet sind. Die Anzeigemittel zur Darstellung des Meßergebnisses können dabei sowohl optischer als auch akustischer Natur sein und dem Benutzer der Vorrichtung unmittelbar das aktuelle Meßergebnis mitteilen. Vorzugsweise sind auch die Auswertemittel in das Handstück integriert.

Durch die Ausgestaltung der optischen Untersuchungsvorrichtung als autonom arbeitendes Handstück ist der Benutzer der Vorrichtung nicht mehr in seinem Bewegungsspielraum eingeschränkt. Die Handhabung der Vorrichtung ist somit im Vergleich zu den bekannten Vorrichtungen, bei denen das Handstück über einen Versorgungsschlauch mit einer Konsole verbunden ist, deutlich verbessert.

Gemäß einer vorteilhaften Weiterbildung dieses Erfindungsgedankens kann vorgesehen sein, dass die von der Untersuchungsvorrichtung erstellten Meßergebnisse zur Ergänzung oder Weiterverarbeitung drahtlos an eine externe Darstellungs- und/oder Auswerteeinheit übermittelt werden. Bei dieser externen Einheit kann es sich beispielsweise um einen PC innerhalb einer zahnärztlichen Praxis handeln, in dem automatisch die während der Untersuchung erstellten Meßergebnisse abgelegt und dem entsprechenden Patienten zugeordnet werden. Ferner kann diese externe Einheit zur zusätzlichen Darstellung des Meßergebnisses - beispielsweise anhand eines Zahnschemas - verwendet werden. Die Übermittlung der Signale kann dabei in Form elektromagnetischer Signale, mittels Ultraschall oder anderer bekannter drahtloser Übertragungstechniken erfolgen.

Ein zweiter Aspekt der vorliegenden Erfindung befaßt sich mit der Problematik der Reinigung bzw. Sterilisierung der Untersuchungsvorrichtung. Wie bereits oben erwähnt wurde, sollte eine einfache Reinigung möglich sein, da in zahnärztlichen Praxen hohe Hygieneanforderungen bestehen. Da zumindest der vordere Teil der Untersuchungsvorrichtung in den Mundraum des Patienten gelangt, sollte insbesondere die Möglichkeit bestehen, diesen Bereich zu sterilisieren.

Gemäß einem zweiten Aspekt der vorliegenden Erfindung wird deshalb vorgeschlagen, das Handstück, in dem die Elemente der optischen Untersuchungsvorrichtung angeordnet sind, zumindest in seinem vorderen Bereich mit einer Hülse zu versehen, welche abnehmbar ist und aus einem sterilisierbarem Material besteht. Da bei dieser Lösung lediglich die Hülse sowie die zur Übermittlung der Anregungs- und Antwortstrahlung erforderliche Untersuchungssonde in Kontakt mit dem Patienten gelangen, können nach einer Untersuchung die Sonde und die Hülse von dem Handstück abgenommen und getrennt sterilisiert werden, wobei insbesondere die zur Ansteuerung der Lichtquelle und Auswertung der Antwortstrahlung erforderlichen elektronischen Komponenten der Vorrichtung in dem nicht zu reinigenden Bereich des Handstücks verbleiben und damit nicht belastet werden.

Entsprechend einer vorteilhaften Weiterbildung dieses zweiten erfindungsgemäßen Gedankens kann die Hülse Teile eines Schaltelements zum Aktivieren der Vorrichtung bzw. der Mittel zum Erzeugen der Anregungsstrahlung aufweisen. Insbesondere kann es sich bei dem Schaltelement um einen Ringschalter handeln, der eine im Inneren des Handstücks verlaufende elektrische Leitung aufweist, welche im Bereich des Ringschalters unterbrochen ist. Die abnehmbare Hülse weist dann im Bereich der Unterbrechung ein betätigbares Element mit einem aus einem leitfähigen Material bestehenden Überbrückungselement auf, über welches die Leitung geschlossen und damit das Handstück bzw. die Mittel zur Erzeugung der Anregungsstrahlung aktiviert werden können. Vorzugsweise handelt es sich bei dem betätigbaren Element um eine aus einem flexiblen Material bestehende Schaltkappe.

Die abnehmbare Hülse ist vorzugsweise von der Vorderseite her auf das Handstück aufsetzbar, wobei insbesondere vorgesehen sein kann, dass die Hülse nur dann aufgeschoben bzw. abgenommen werden kann, wenn die zur Übermittlung der Anregungs- und Antwortstrahlung vorgesehene Diagnosesonde von dem Handstück abgenommen ist. Auch diese Sonde ist vorzugsweise an dem vorderen Handstückende lösbar befestigt, insbesondere verrastet und kann drehbar gelagert sein. Dabei kann ferner auch vorgesehen sein, unterschiedlich gestaltete Diagnosesonden bereitzustellen, welche das Licht der Anregungs- und Antwortstrahlung entsprechend dem Bereich, der gerade untersucht werden soll, in jeweils bestimmter Weise ein- und auskoppeln. Hierdurch wird die Möglichkeit eröffnet, die erfindungsgemäße Untersuchungsvorrichtung z.B. sowohl zur Untersuchung von Kauflächen als auch zur Untersuchung in Zahnfleischtaschen und Zahnzwischenräumen zu verwenden.

Ein dritter Aspekt der vorliegenden Erfindung befaßt sich mit der Problematik, die zur Erfassung der von der Zahnoberfläche zurückgeworfenen Antwortstrahlung erforderlichen Elemente möglichst kompakt zu gestalten, so dass diese platzsparend in das Handstück integriert werden können.

Dabei ist gemäß dem dritten Aspekt der vorliegenden Erfindung vorgesehen, eine zum Erfassen der Antwortstrahlung vorgesehene Fotodiode innerhalb eines kleinen Gehäuses anzuordnen, welches in das Handstück eingesetzt ist. Die Übermittlung der Antwortstrahlung zu der Fotodiode erfolgt dabei über einen Lichtleiter, an dessen einem Ende die Fotodiode angeordnet ist. Um eine optimale Einkopplung des von dem Lichtleiter übertragenen Lichts in die Fotodiode zu ermöglichen, kann dabei das Gehäuse eine Öffnung aufweisen, in welche der Lichtleiter mündet, wobei an dem Gehäuse insbesondere ein Schnappverschluß zum Befestigen des Lichtleiters angeordnet sein kann. Vorzugsweise ist innerhalb des Gehäuses noch ein zwischen dem Lichtleiterende und der Fotodiode angeordnetes Filter vorgesehen, welches nur die zur Auswertung der Antwortstrahlung erforderlichen Wellenlängen durchläßt. Diese kompakte Anordnung ermöglicht eine sehr effektive Auswertung der auf die Bestrahlung mit der Anregungsstrahlung hin entstehenden Antwortstrahlung, wobei gleichzeitig eine weitgehende Miniaturisierung erzielt wird.

Andere Weiterbildungen und vorteilhaften Ausgestaltungen der erfindungsgemäßen Vorrichtung sind Gegenstand der Unteransprüche. Sie werden im Zusammenhang mit der Beschreibung mehrerer Ausführungsbeispiele der vorliegenden Erfindung anhand der beiliegenden Zeichnungen näher erläutert. Dabei zeigen:
- Fig. 1: eine erfindungsgemäße zahnärztliche optische Untersuchungsvorrichtung in Form eines Handstücks;
- Fig. 2a: die Ausgestaltung und Anordnung der wesentlichen Komponenten zur optischen Kariesdiagnose;
- Fig. 2b und 2c: vergrößerte Darstellungen von Fig. 2a;
- Fig. 3: eine vergrößerte Darstellung eines ersten Ausführungsbeispiels einer Diagnosesonde zum Übermitteln der Anregungs- und Antwortstrahlung;
- Fig. 4: eine zweite Variante einer Diagnosesonde im Schnitt;
- Fig. 5: eine dritte Variante einer Diagnosesonde;
- Fig. 6: ein Schema zur Übermittlung der Anregungs- und Antwortstrahlung gemäß einem ersten Ausführungsbeispiel;
- Fig. 7: eine Darstellung einer erfindungsgemäßen Anordnung und Ausgestaltung der zur Auswertung der Antwortstrahlung vorgesehenen Fotodiode;
- Fig. 8 und 9: zwei weitere Möglichkeiten zur Übermittlung der Anregungs- und Antwortstrahlung;
- Fig. 10: das Handstück bei abgenommener Hülse;
- Fig. 11: die von dem Handstück abnehmbare Hülse;
- Fig. 12 und 13: eine Möglichkeit zur Realisierung eines einfach zu reinigenden Schaltelements zur Aktivierung der Anregungsstrahlung;
- Fig. 14: die erfindungsgemäße Untersuchungsvorrichtung im Teilschnitt;

- Fig. 15: die Untersuchungsvorrichtung in einer weiteren perspektivischen Darstellung;
- Fig. 16: die Anordnung einer zur Stromversorgung der Vorrichtung vorgesehenen Batterie innerhalb des Handstücks;
- Fig. 17 und 18: zwei Darstellungen einer erweiterten Variante der erfindungsgemäßen Vorrichtung, bei der zusätzlich ein Funkmodul zur drahtlosen Übertragung der Meßdaten vorgesehen ist;
- Fig. 19: das an dem Handstück vorgesehene Bedienfeld zur Aktivierung bzw. Einstellung der unterschiedlichen Funktionen;
- Fig. 20: die an dem Handstück vorgesehene Anzeige zur Darstellung der Funktionen und Meßergebnisse und
- Fig. 21: das Bedien- und Benutzungsschema der erfindungsgemäßen Untersuchungsvorrichtung.

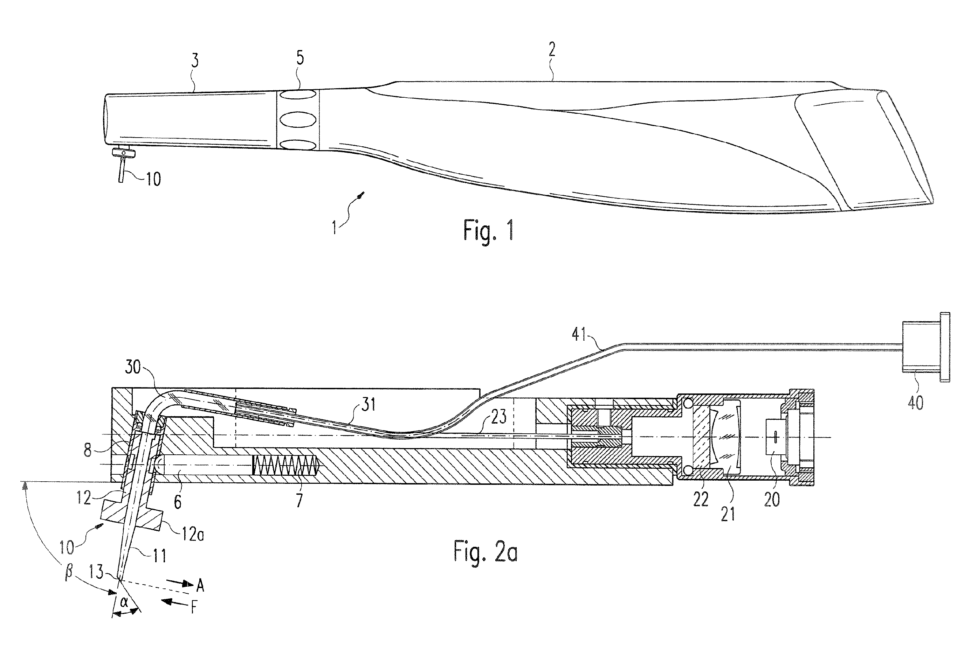

Fig. 1 zeigt die Außenansicht eines ersten Ausführungsbeispiels einer erfindungsgemäßen zahnärztlichen Vorrichtung zum Untersuchen von Zahngewebe, insbesondere zum Erkennen von Karies, Plaque, bakteriellen Befall, Konkrementen oder Zahnstein. Die Vorrichtung ist als Handstück 1 ausgestaltet, welches gemäß dem ersten Erfindungsgedanken vollkommen unabhängig von weiteren externen Konsolen oder Auswerte- und Darstellungseinheiten verwendet werden kann. Der längliche Griffkörper 2 weist hierzu in seinem vorderen Kopfbereich 3 eine seitlich leicht schräg nach unten stehende Sonde 10 auf, welche zur Übermittlung einer Anregungsstrahlung auf den zu untersuchenden Zahngewebebereich sowie zur Übermittlung der von dem Zahn zurückgestrahlten Antwortstrahlung zu einer in dem Handstück 1 angeordneten Auswerteeinheit vorgesehen ist. Die genaue Ausgestaltung und Funktion dieser Sonde 10 wird später noch ausführlich erläutert.

Im vorderen Bereich 3 des Handstücks 1 ist ferner ein Ringschalter 5 vorgesehen, der zum Aktivieren der Anregungsstrahlungsquelle verwendet werden kann. Wie ebenfalls später noch ausführlicher erläutert wird, ist dieser Ringschalter 5 Bestandteil einer von dem Handstück 1 zur Vorderseite hin abnehmbaren Hülse, wodurch ein einfaches Reinigen und Sterilisieren derjenigen Teile des Handstücks 1, die mit dem Patienten in Kontakt gelangen, ermöglicht wird.

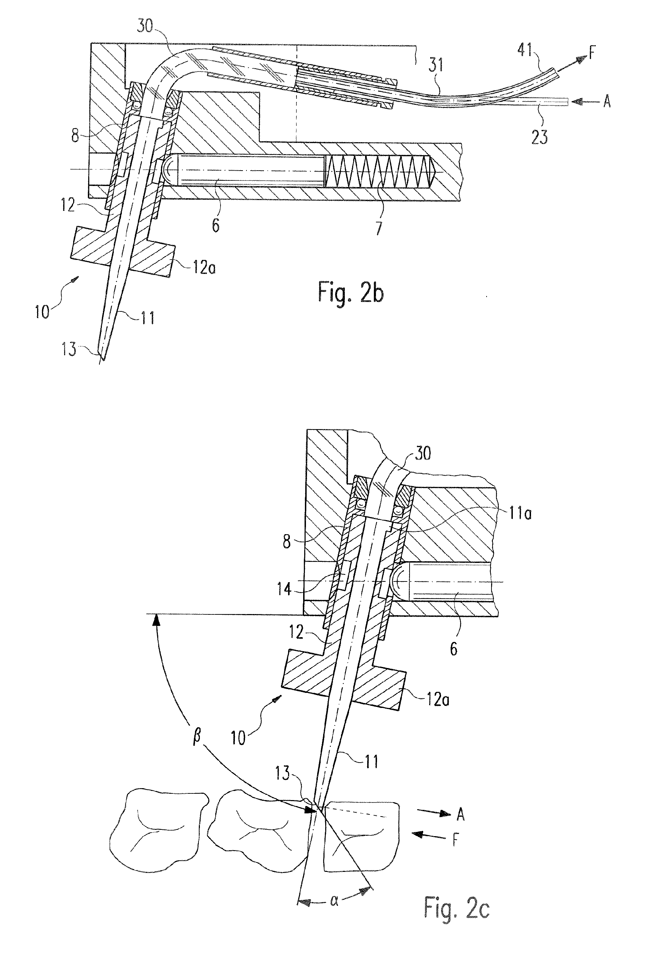

Die Fig. 2a bis 2c zeigen diejenigen Komponenten der Untersuchungsvorrichtung, die zum Erkennen der oben genannten Materialien an einem untersuchten Zahngewebebereich erforderlich sind. Wie bei den bereits bekannten optischen Untersuchungsvorrichtungen erfolgt die Untersuchung dadurch, dass das zu untersuchende Zahngewebe einer Anregungsstrahlung ausgesetzt wird und die auf die Bestrahlung hin entstehende Antwortstrahlung erfaßt und ausgewertet wird. Die wesentlichen Komponenten des Handstücks 1 sind somit zum einen eine Lichtquelle zur Erzeugung der Anregungsstrahlung, Auswertemittel zum Auswerten der Antwortstrahlung sowie Mittel zum Übertragen der Anregungsstrahlung zu dem zu untersuchenden Bereich sowie zum Übertragen der Antwortstrahlung zu den Auswertemitteln.

Bei dem dargestellten Handstück 1 wird die Lichtquelle zur Erzeugung der Anregungsstrahlung durch eine Laserdiode 20 gebildet, welche ein nahezu monochromatisches Licht erzeugt. Insbesondere kann die Anregungsstrahlung im Bereich zwischen 600 nm und 670 nm, vorzugsweise bei ca. 655 nm liegen, da bei einer derartigen Wellenlänge der bestmögliche Kompromiß zwischen der Ausgangsleistung der Laserdiode 20 und dem spektralen Unterschied zwischen der Anregungsstrahlung und der von der Zahnoberfläche zurückgeworfenen Antwortstrahlung erzielbar ist. Anzumerken ist, dass die Funktion des erfindungsgemäßen Handstücks hier am Beispiel einer Fluoreszenzdiagnose erläutert wird, bei der die an der Zahnoberfläche als Reaktion auf die Bestrahlung hin entstehende Fluoreszenzstrahlung bewertet wird. Alternativ dazu bestünde allerdings auch die Möglichkeit, andere Wellenlängen für die Anregungs- und/oder Antwortstrahlung zu verwenden oder im Rahmen einer sog. Reflexionsspektrometrie zur untersuchen, welche Wellenlängen von der Zahnoberfläche in welcher Weise reflektiert werden.

Mit Hilfe einer der Laserdiode 20 vorgeschalteten Linse 21 sowie einem optischen Filter 22, der das von der Laserdiode 20 abgegebene Licht nochmals auf den gewünschten Wellenlängenbereich eingrenzt, wird dann eine Anregungsstrahlung A erzeugt und in einen ersten Lichtleiter 23 eingekoppelt. Bei diesem Lichtleiter 23 kann es sich um eine einzelne Lichtfaser mit einem Durchmesser von ca. 0,5 mm handeln, es bestünde allerdings auch die Möglichkeit, den Lichtleiter 23 aus einer Vielzahl von einzelnen Lichtleiterfasern zu bilden. An seinem vorderen Ende grenzt der Lichtleiter 23 an einen gekrümmten Faserstab 30 aus einem ebenfalls lichtleitenden Material an, durch den die Anregungsstrahlung umgelenkt und in die Stirnseite der Diagnosesonde 10 eingekoppelt wird.

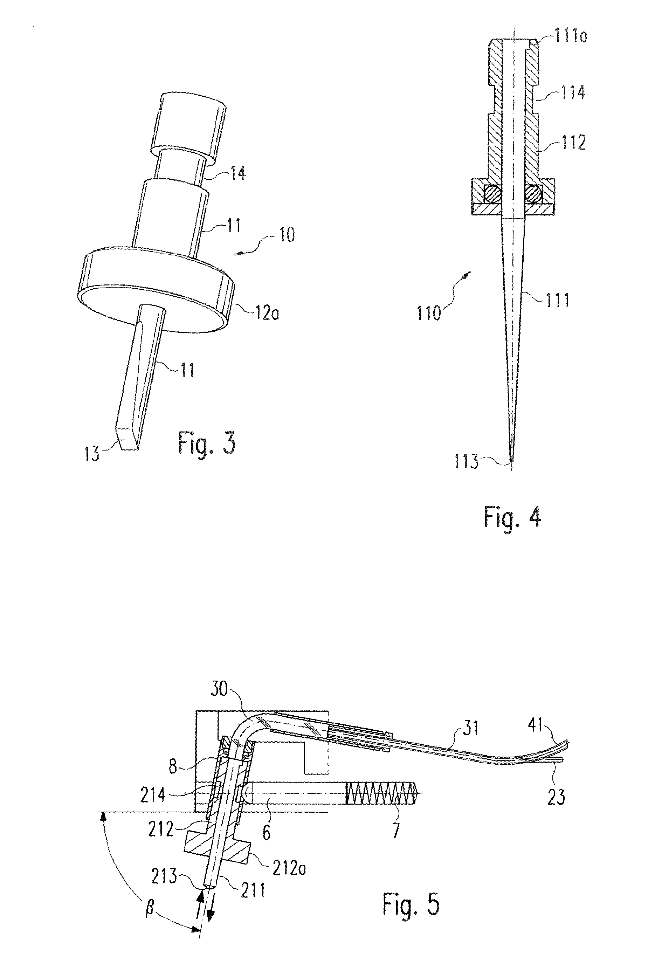

Die genaue Ausgestaltung der Diagnosesonde 10 kann der perspektivischen Darstellung in Fig. 3 entnommen werden. Es handelt sich hierbei um eine Sonde zur Untersuchung von Zahnzwischenräumen. Wesentliches Element der Sonde 10 ist ein länglicher Lichtkeil 11 aus einem transparenten Material, an dessen unterem Ende die Anregungsstrahlung A ausgekoppelt und auf den zu untersuchenden Zahnbereich gerichtet wird. Als Material für den Lichtkeil 11 kann beispielsweise Kunststoff oder Saphir verwendet werden, wobei Kunststoff hinsichtlich der geringeren Bruchgefahr und Herstellungskosten vorteilhaft ist, allerdings Nachteile im Hinblick auf Abnutzungserscheinungen und die daraus resultierende Lebensdauer aufweist.

Wie insbesondere der Darstellung in Fig. 2c entnommen werden kann, soll das Licht seitlich zur Längsachse aus dem Lichtkeil 11 ausgekoppelt werden, um eine Untersuchung der Zahnzwischenräume zu ermöglichen. Hierzu ist das vordere Ende des aus einem transparenten Material bestehenden Lichtkeils 11 mit einer Schräge 13 versehen, die mit der Längsachse des Lichtkeils einen Winkel α von ca. 40 bis 45° einschließt. Das von oben einfallende Licht wird dann an dieser Schräge 13 totalreflektiert und seitlich aus dem Lichtkeil 11 ausgekoppelt. Zusätzlich oder alternativ könnte die Schräge 13 auch verspiegelt sein, um die Umlenkung des Licht zu erzielen.

Die Rückübermittlung der auf die Bestrahlung hin an der Zahnoberfläche entstehenden Antwortstrahlung F erfolgt in ähnlicher Weise in umgekehrter Richtung. Zunächst fällt die Antwortstrahlung seitlich in den Lichtkeil 11 ein und wird wiederum an der Schräge 13 reflektiert und somit zur Stirnseite der Sonde 10 gelenkt und in den Faserstab 30 eingekoppelt. Von dem Ende des Faserstabs 30, der wiederum vorzugsweise aus einer Vielzahl von Einzelfasern mit einem Durchmesser von 0,1 mm besteht und insgesamt einen Durchmesser von ca. 1,4 mm aufweist, wird dann die Antwortstrahlung in ein Lichtfaserbündel 31 eingekoppelt, welches zum einen aus der bzw. den Anregungsstrahlungsfaser(n) 23 für die Anregungsstrahlung A und zum anderen aus einer Detektionsfaser 41 zur Übermittlung der Antwortstrahlung F besteht.

Über die Detektionsfaser 41, die vorzugsweise einen Durchmesser von 0,25 mm aufweist, erfolgt dann die Weiterleitung zu einer Erfassungseinrichtung 40, deren näherer Aufbau später noch beschrieben wird. Die Aufgabe dieser Erfassungseinrichtung 40 ist es, die von der Zahnoberfläche zurückgestrahlte Antwortstrahlung zu erfassen, zu analysieren und auf Basis des Meßergebnisses zu beurteilen, ob eine der eingangs genannten fluoreszierenden Substanzen an der untersuchten Zahnoberfläche vorhanden ist oder nicht.

Zu der Diagnosesonde 10 ist zu anzumerken, dass diese in dem Kopfbereich 3 des Handstücks 1 um 360° drehbar gelagert ist, so dass das Handstück 1 sehr flexibel an die zu untersuchenden Zähne herangeführt werden kann. Die Sonde 10 ist dabei mit dem Kopfbereich 3 des Handstücks 3 verrastet und kann in sehr einfacher Weise - z.B. zu Reinigungszwecken oder zum Ersetzen durch eine andere Sonde - entnommen werden. Hierzu sind in dem Kopfbereich 3 des Handstücks 1 zunächst eine zylinderförmige Führung 8 für die Sonde 10 sowie ein Raststift 6 vorgesehen, der mit Hilfe einer Feder 7 gegen die Sonde 10 drückt und diese damit in der eingesetzten Position innerhalb des Führung 8 hält. Das vordere Kugelsegment des Raststifts 6 greift dabei in eine umlaufende Ausnehmung 14 eines Halters 12 für den Lichtkeil 11 ein, so dass die Sonde 10 immer auf Anschlag gedrückt wird und sicher innerhalb des Handstücks 1 aber zugleich drehbar gelagert ist. Eine Drehung der Sonde 12 von Hand wird dabei durch einen scheiben- oder ringförmigen Aufsatz 12a an dem Halter 12 erleichtert, der von einem Benutzer des Handstücks 1 mit den Fingern leicht gegriffen und verdreht werden kann. Der Halter 12 selbst weist eine längliche Bohrung auf, in welche der Lichtkeils 11 eingesetzt ist, wobei die Möglichkeit besteht, den Lichtkeil 11 auszuwechseln. Ein definierte Anordnung bzw. Orientierung des Lichtkeils 11 innerhalb des Halters 12 ist durch eine Nase 11a sichergestellt, welche mit einer entsprechenden Ausnehmung in dem Halter 12 zusammenwirkt.

Anzumerken ist ferner, dass die Diagnosesonde 10 gegenüber der Handstücklängsachse nicht in einem rechten Winkel, sondern vorzugsweise leicht schräg in einem Winkel β von ca. 80° gehalten wird. Es hat sich herausgestellt, dass hierdurch eine besonders ergonomisch günstige Handhabung der erfindungsgemäßen Untersuchungsvorrichtung erzielt wird.

Die lösbare Halterung der Sonde 10 bringt zum einen den Vorteil mit sich, dass die Sonde 10 nach jeder Untersuchung entfernt und getrennt von den übrigen Bestandteilen des Handstücks 1 gereinigt und desinfiziert werden kann. Zum anderen besteht allerdings auch der Vorteil, dass die Sonde 10 leicht ausgetauscht und durch eine anders gestaltete Sonde ersetzt werden kann. Es besteht hierdurch die Möglichkeit, unterschiedlich geformte Sonden bereitzustellen, die je nach Ort bzw. Oberflächengestaltung der zu untersuchenden Zahnstelle ausgestaltet sein können.

Die Fig. 4 und 5 zeigen beispielhaft zwei weitere bei dem erfindungsgemäßen Diagnosesystem zum Einsatz kommende Sonden. Bei der in Fig. 4 dargestellten Sonde 110 handelt es sich um eine sogenannte Parosonde, die zur Untersuchung von Zahnfleischtaschen und insbesondere zum Lokalisieren von subgingivalen Konkrementen an Zahnwurzeln vorgesehen ist. Im Gegensatz zu der in den vorherigen Figuren dargestellten Sonde 10 zur Untersuchung des Approximalraums bzw. Zahnzwischenraums weist die Parosonde 110 keine Abschrägung an der Sondenspitze auf, sondern ist lediglich am äußersten Ende gerade abgeflacht. Hierdurch wird ein sehr schlanker Lichtaustritt mit einem kleinen Durchmesser erzielt, so dass die Anregungsstrahlung sehr konzentriert auf eine zu untersuchende Stelle gerichtet werden kann. Ein Lichtaustritt am Umfang der Sonde 110 hingegen ist nicht vorgesehen. Die Parosonde 110 besteht somit wiederum aus einem länglichen Lichtkeil 111 aus einem lichtleitendem Material mit einer vorderen Lichtaustrittsspitze 113, wobei der Lichtkeil 111 durch einen Halter 112 mit einem scheibenförmigen Aufsatz 112a gehalten ist, welcher im hinteren Bereich wieder die umlaufende Ausnehmung 114, über die eine Verrastung mit dem Handstück erzielt wird, aufweist.

Eine dritte Sonde 210 zur Durchführung von Untersuchungen im Fissurenbereich - also zur Untersuchung der Kauflächen von Zähnen oder von Glattflächen bzw. Zahnaußenflächen - ist in Fig. 5 dargestellt. Zentrales Element dieser dritten Sonde 210 ist ein zylinderförmiger Lichtstab 211, der in seinem vorderen Endbereich ein kegelstumpfförmiges Ende 213 aufweist, über das Licht in Längsrichtung der Sonde 210 abgegeben und in umgekehrter Richtung eingekoppelt wird. Auch diese dritte Sonde 210 weist die zur lösbaren Verrastung mit dem Handstück 1 erforderliche Ausnehmung 214 sowie den Halter 212 mit dem scheibenförmigen Aufsatz 212a auf. Anzumerken ist allerdings, dass bei den Sonden der Fig. 4 und 5 die Drehbarkeit nicht unbedingt erforderlich ist, da ein Lichtaustritt bzw. Lichteintritt ohnehin immer in Richtung der Längsachse der Sonde 110 bzw. 210 erfolgt.

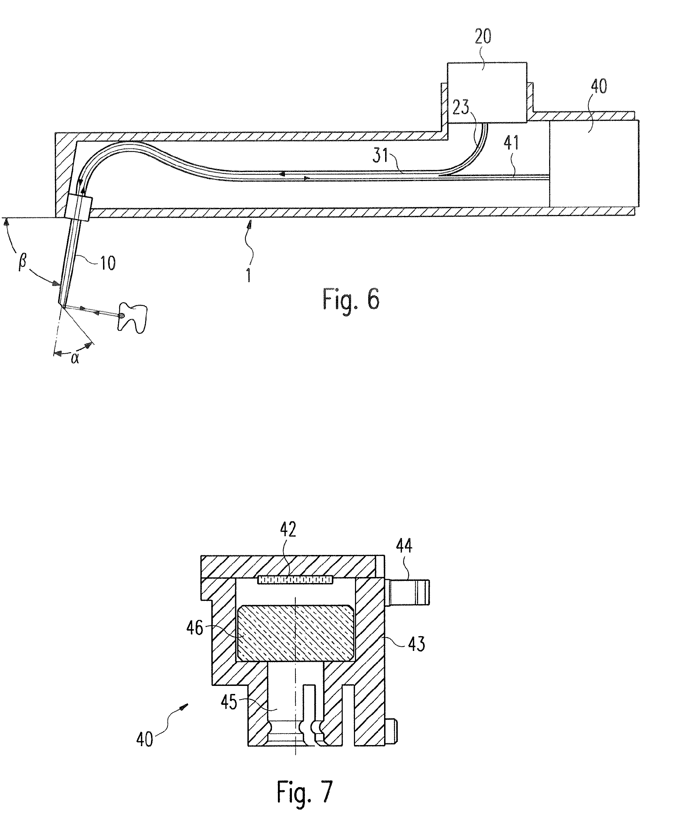

Bei dem in den Fig. 2a bis 2c dargestellten Ausführungsbeispiel erfolgte die Übertragung der Anregungs- und Antwortstrahlung A bzw. F im vorderen Endbereich des Handstücks 1 mit Hilfe eines gekrümmten Faserstabs 30, an dessen handstückseitigen Ende sich die verschiedenen Lichtleiterbündel 23 bzw. 41 für die Anregungsstrahlung und die Antwortstrahlung anschlossen. Wie in Fig. 6 dargestellt ist, besteht allerdings auch die Möglichkeit, auf diesen Faserstab 30 zu verzichten und statt dessen die beiden Bündel 23 für die Anregungsstrahlung und 41 für die Antwortstrahlung zu einem gemeinsamen Faserbündel 31 zusammenzufassen, welches sich bis zu dem Stirnbereich der Sonde 10 erstreckt und direkt Licht in diese einkoppelt bzw. Licht aus dieser auskoppelt. Das gemeinsame Faserbündel 31 besteht somit zum einen aus Fasern für die Übermittlung der Anregungsstrahlung A sowie zum anderen aus Fasern für die Übermittlung der Antwortstrahlung F. Insbesondere kann vorgesehen sein, die Fasern für die Anregungsstrahlung A zentral anzuordnen und konzentrisch mit den Fasern für die Antwortstrahlung F zu umgeben.

Sowohl bei dem Ausführungsbeispiel der Fig. 2a bis 2c als auch bei der in Fig. 6 dargestellten Variante mündet allerdings ein Lichtleiter 41 in eine Vorrichtung 40 zur Erfassung der Antwortstrahlung, deren nähere Ausgestaltung nunmehr anhand von Fig. 7 erläutert werden soll.

Zentrales Element der Erfassungseinheit 40 ist dabei eine Fotodiode 42, welche die Antwortstrahlung F erfaßt und in ein elektrisches Signal entsprechend der Intensität der Antwortstrahlung F umsetzt. Die Höhe der Intensität der Antwortstrahlung F gibt letztendlich darüber Auskunft, ob an dem untersuchten Zahngewebebereich fluoreszierende Materialien vorhanden sind oder nicht.

Da es sich bei dieser Fotodiode 42 bzw. dem zur Umsetzung der auftreffenden Anwortstrahlung in ein elektrisches Signal verantwortlichen Silicium-Chip um ein verhältnismäßig sensibles Bauteil handelt, sollte diese gegen externe Einflüsse und Erschütterungen möglichst gut geschützt sein. Gemäß dem in Fig. 7 dargestellten bevorzugten Ausführungsbeispiel ist daher die Fotodiode 42 in ein Gehäuse 43 integriert, welches als ganzes in dem Handstück 1 angeordnet ist. Ein seitlich von dem Gehäuse 43 abstehender Zapfen 44 dient zur Verrastung auf einer Platine innerhalb des Handstücks 1.

Damit das durch das Faserbündel 41 übermittelte Licht der Antwortstrahlung F möglichst effektiv ausgewertet werden kann, ist erforderlich, das Licht möglichst genau auf die Fotodiode 42 zu richten. Um dies zu gewährleisten, ist das vordere Ende des Gehäuses 43 mit einer zylinderförmigen Öffnung 45 versehen, in die das Lichtleiterbündel eingeschoben wird. Durch die Öffnung 45 wird das Bündel so gehalten, dass das Licht unmittelbar auf die Fotodiode 42 gerichtet wird. Darüber hinaus ist an der Öffnung 45 ein Schnappverschluß vorgesehen, durch den das Faserbündel 41 auch fest in der gewünschten Position gehalten wird. Schließlich ist in das Gehäuse 43 noch ein Filter 46 integriert, welches die für die Auswertung der Antwortstrahlung F nicht relevanten Wellenlängenbereiche ausfiltert. Es wird somit sichergestellt, dass durch die Fotodiode 42 nur solches Licht erfasst und ausgewertet wird, welches auch für die Diagnose von fluoreszierenden Materialien relevant ist. Bei dem oben angesprochenen Beispiels der Fluoreszenzdiagnostik mit einer Anregungswellenlänge von ca. 655nm ist das Filter 46 beispielsweise derart ausgelegt, dass lediglich Strahlung mit Wellenlängen oberhalb von 680nm durchgelassen wird. Die in Fig. 7 dargestellte Anordnung ist somit nicht nur äußerst kompakt sondern gewährleistet gleichzeitig eine möglichst effektive und genaue Auswertung der Antwortstrahlung F.

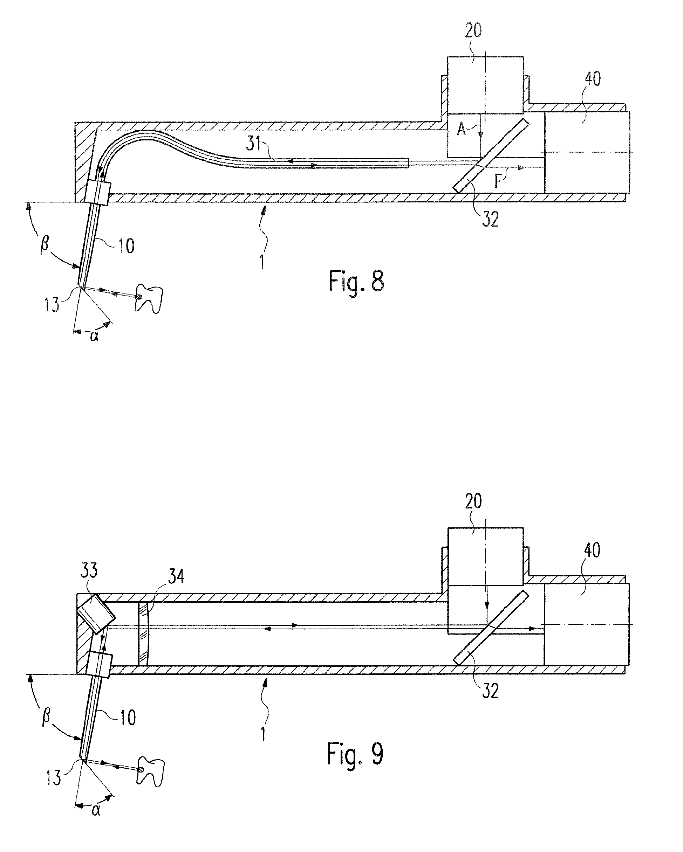

Die Fig. 8 und 9 zeigen in Ergänzung zu den Figuren 2a-c und 7 zwei weitere Varianten zur Übermittlung der Anregungs- und Antwortstrahlung. Dabei ist bei der Variante gemäß Fig. 8 wiederum zunächst ein Faserbündel 31 vorgesehen, welches sowohl zur Übermittlung der Anregungs- als auch der Antwortstrahlung vorgesehen ist. Das Faserbündel 31 spaltet sich dann allerdings nicht in zwei getrennte Bündel für die verschiedenen Strahlungen auf, sondern ist mit seinem rückwärtigen Ende auf einen Teilerspiegel 32 bzw. Strahlteiler gerichtet, der teillichtdurchlässig und für die Ein- und Auskopplung der Anregungs- und Antwortstrahlung verantwortlich ist. Der gegen die optische Achse des rückwärtigen Endes des Faserbündels 31 geneigt angeordnete Teilerspiegel 32 besteht dabei aus einem Material, welches zum einen die von der Fotodiode 20 stammende Anregungsstrahlung A reflektiert und zum anderen die interessierende Antwortstrahlung F möglichst ungehindert in Richtung der Erfassungseinheit 40 durchläßt. Selbstverständlich können die Positionen der Fotodiode 20 und der Erfassungseinheit 40 auch getauscht werden, sofern der Teilerspiegel 32 entsprechend beschichtet ist, also für die entsprechenden Wellenlängen in geeigneter Weise durchlässig bzw. reflektierend ist.

Bei der Variante gemäß Fig. 9 hingegen wird auf ein gemeinsames Faserbündel zur Übertragung der Strahlungen vollständig verzichtet und statt dessen das Licht der Anregungs- und Antwortstrahlung mit Hilfe von Spiegeln und Linsen auf die Sonde hin bzw. in umgekehrter Richtung gelenkt. Das bei der Variante in Fig. 8 vorgesehene Faserbündel 31 wird hierbei durch einen Umlenkspiegel 33 im Kopfbereich des Handstücks 1 ersetzt, der das Licht in geeigneter Weise umlenkt. Mit Hilfe einer ebenfalls im Kopfbereich angeordneten Fokussierlinse 34 wird sichergestellt, dass das Licht auch in gewünschter gebündelter Weise auf die verschiedenen optischen Elemente auftrifft. Die Ein- und Auskopplung der Anregungs- und Antwortstrahlung erfolgt wiederum mit Hilfe des im rückwärtigen Bereich des Handstücks angeordneten Teilerspiegels 32.

Wie bereits eingangs erwähnt wurde, ist bei Untersuchungsvorrichtungen, die in Kontakt mit einem Patienten gelangen, von größter Wichtigkeit, dass diese auch effektiv gereinigt und sterilisiert werden können. Da gemäß der vorliegenden Erfindung sämtliche Komponenten der Untersuchungseinrichtung in dem Handstück 1 angeordnet sind, ergibt sich nunmehr die Schwierigkeit, dass die elektronischen Komponenten zur Steuerung und Auswertung der Antwortstrahlung nicht zu großen Belastungen ausgesetzt sein dürfen. Da bei den üblicherweise zum Einsatz kommenden Heißluftsterilisationsverfahren die elektronischen Bauteile beschädigt werden könnten, müssen Maßnahmen getroffen werden, mit denen dies verhindert wird.

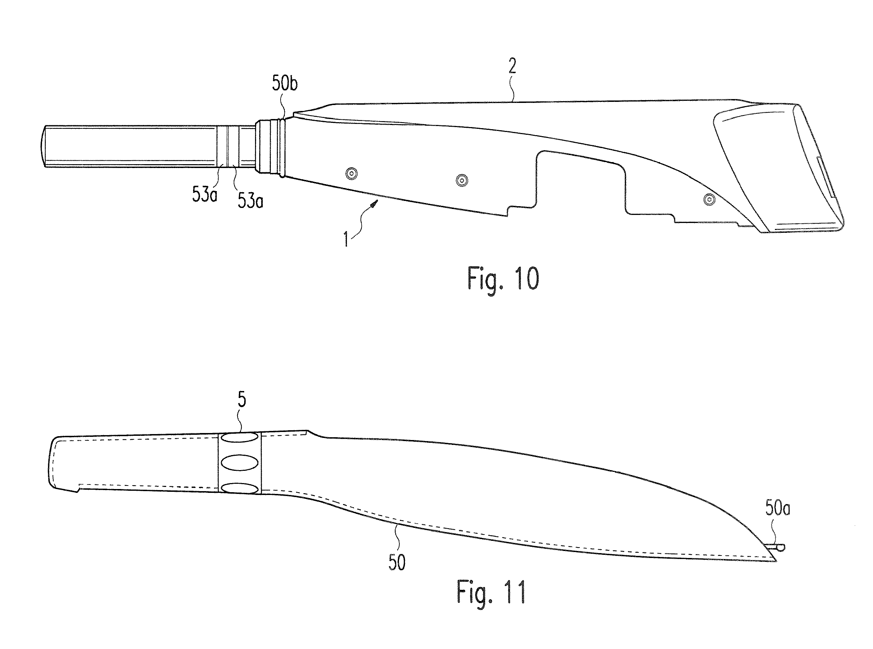

Die erfindungsgemäße Lösung dieses Problems besteht darin, das Handstück 1 mit einer Hülse zu versehen, die von dem Handstück entfernt und separat sterilisiert werden kann, wobei die Hülse selbst derjenige Teil ist, der mit dem Patienten in Kontakt gerät und möglichst wenig elektronische Komponenten beinhaltet. Diese Lösung soll anhand der Fig. 10 bis 13 nunmehr näher erläutert werden.

Fig. 10 zeigt dabei das Handstück mit der bereits abgenommenen Hülse 50, die im abgenommenen Zustand in Fig. 11 dargestellt ist. Die Hülse 50 umhüllt dabei insbesondere den vorderen Endbereich des Handstücks 1 vollständig, so dass sichergestellt ist, dass nur die Hülse 50 in Kontakt mit dem Patienten gerät, nicht jedoch die inneren Komponenten der Untersuchungsvorrichtung. Sobald die Diagnosesonde von dem Handstück 1 abgenommen wurde, kann die Hülse 50 dann von der Vorderseite her abgezogen werden bzw. auf den Handstückkörper 2 aufgeschoben werden, wobei mit Hilfe eines Raststifts 50a am rückwärtigen Ende der Hülse 50 ein sicherer Halt zwischen Hülse 50 und Handstückkörper 2 erzielt wird. Der Halt zwischen beiden Elementen wird ferner durch einen O-Ring 50b verbessert, der im mittleren Bereich des Handstückkörpers 2 angeordnet ist und darüber hinaus auch für eine Abdichtung des rückwärtigen Handstückbereichs sorgt.

Wesentlich ist, dass die Hülse 50 aus einem Material besteht, welches einfach zu reinigen und zu sterilisieren ist. Ferner sind die wesentlichen elektronischen Komponenten des erfindungsgemäßen Handstücks 1 - wie bereits erwähnt - nicht in der Hülse 50 sondern in dem verbleibenden Bereich des Handstücks 1 angeordnet, der nicht sterilisiert werden muß.

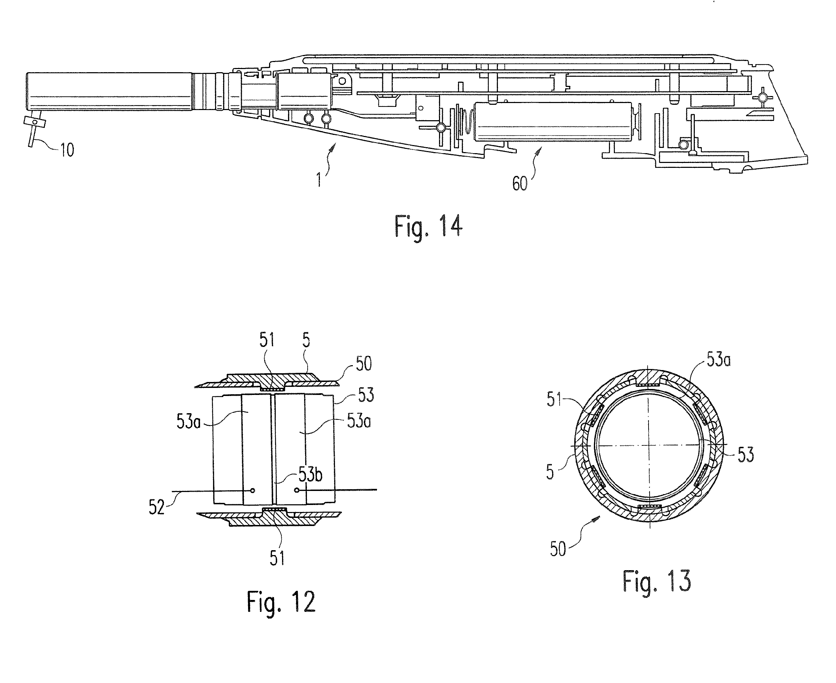

Da das Handstück 1 während der Benutzung allerdings vorwiegend in seinem vorderen Bereich durch die Finger des Benutzers gehalten wird, sollte die Möglichkeit bestehen, die Lichtquelle für die Anregungsstrahlung griffgünstig aktivieren und damit die Untersuchung starten zu können, ohne hierzu den Griff verändern zu müssen. Es ist dementsprechend ein Ringschalter 5 vorgesehen, der auch bei aufgesetzter Hülse 50 betätigt werden kann und trotz allem durch den Sterilisierungsvorgang nicht beschädigt wird. Die erfindungsgemäße Ausgestaltung des Ringschalters ist in den Fig. 12 und 13 näher dargestellt.

Im wesentlichen besteht der Schalter aus zwei Bestandteilen, die zum einen an bzw. in der Hülse 50 und zum anderen im Inneren des Handstücks angeordnet sind. Wesentlicher Bestandteil des Schalters im Innenraum des Handstücks 1 ist eine elektrische Leitung 52, die an der Stelle des Ringschalters unterbrochen ist. Die Leitungen enden hierbei jeweils auf einer zylinderförmigen Kunststoffhülse 53, die zwei voneinander beabstandet angeordnete umlaufende Leiterbahnen 53a (welche auch in Fig. 10 dargestellt sind) aufweist, welche durch einen Spalt 53b voneinander getrennt sind. Durch die Herstellung eines elektrischen Kontakts zwischen den beiden Leiterbahnen 53a kann die Leitung 52 geschlossen und somit ein Aktivierungssignal für die Untersuchungseinrichtung erzeugt werden. Hierzu sind in der Hülse 50 mehrere plattenförmige Kontakte 51 vorgesehen, welche Bestandteile einer Schaltkappe in Form eines flexiblen Rings 5 sind, der Bestandteil der Hülse 50 ist und im aufgesetzten Zustand der Hülse 50 auf dem Handstück 1 im Bereich der Leiterbahnen 53a angeordnet ist. Durch ein Zusammendrücken des Ringbereichs 5 der Hülse 50 wird somit zumindest ein Kontaktierungselement 51 auf die beiden Leiterbahnen 53a gedrückt und stellt die elektrische Verwendung her, durch die dann die Untersuchungseinrichtung aktiviert wird.

Anstelle der in den Fig. 12 und 13 dargestellten auf die Innenseite der Hülse 50 aufgebrachten separaten Kontaktpillen 51 bestünde auch die Möglichkeit, leitfähige Elemente in die aus einem isolierenden Material bestehende Hülse 50 zu integrieren, beispielsweise einzuvulkanisieren. Als geeignetes Material würde sich hierfür beispielsweise Carbon anbieten, welches entweder in Form von einzelnen Kontaktierungsbereichen oder als ringförmige Kontaktierungsstruktur in die Hülse 50 eingearbeitet werden könnte. Ferner wäre auch denkbar, die Hülse 50 zumindest im Bereich des Ringschalters 5 vollständig aus einem flexiblen aber leitfähigen Material zu bilden.

Wesentlich bei der erfindungsgemäßen Ausgestaltung ist, dass lediglich die Kontaktierungselemente 51 gemeinsam mit der Hülse 50 sterilisiert werden müssen. Diese können allerdings aus einem Material bestehen, welches relativ unempfindlich gegenüber den hohen Temperaturen einer Heißluftsterilisation ist, so dass somit ein Schaltelement zum Aktivieren der Untersuchungsvorrichtung erhalten wird, welches trotz allem gut sterilisierbar ist. Die weiteren Komponenten des Schalters sowie der Untersuchungsvorrichtung insgesamt hingegen müssen nicht sterilisiert werden, so dass eine Beschädigung dieser vermieden wird.

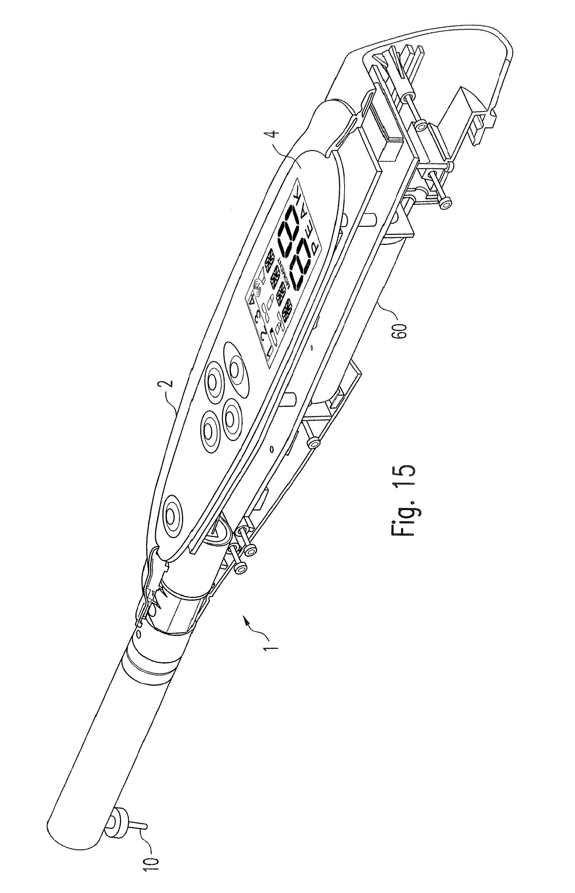

Die Fig. 14 und 15 zeigen das erfindungsgemäße Handstück in seitlicher bzw. perspektivischer Darstellung im Teilschnitt. Wie den Darstellungen entnommen werden kann, ist im rückwärtigen Bereich des Handstückkörpers eine Batterieaufnahme zur Stromversorgung der erfindungsgemäßen Untersuchungsvorrichtung vorgesehen. Am oberen Ende des rückwärtigen Bereichs des Handstückkörpers 2 befindet sich ferner ein Bedien- und Anzeigefeld 4, welches später noch erläutert wird. Das Handstück 1 weist ferner auch einen Lautsprecher auf, über den das aktuelle Meßergebnis akustisch dargestellt werden kann. Beispielsweise könnte hiermit die Intensität der gemessenen Antwortstrahlung durch ein sich in der Frequenz und/oder Lautstärke veränderndes Signal dargestellt werden.

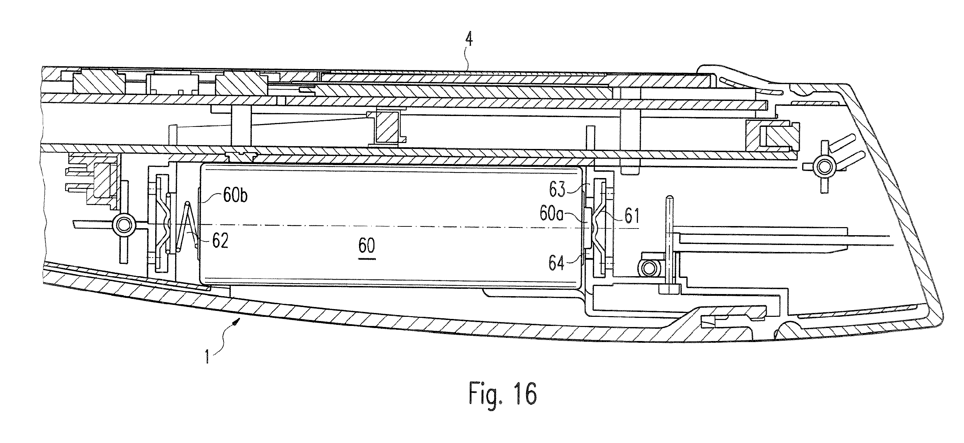

Die Aufnahme für die Batterie 60 bzw. Batterien zur Stromversorgung ist derart gestaltet, dass ein versehentliches verkehrtes Einlegen der Batterien 60 verhindert wird. Dieser Verpolungsschutz ist in Fig. 16 dargestellt und weist als wesentliches Element in dem Bereich, der zur Kontaktierung des positiven Pols 60a der Batterie 60 vorgesehen ist, eine Anlagefläche 63 auf, die eine kreisförmige Ausnehmung 64 enthält. Der positive Pol 60a kann durch diese Ausnehmung 64 durchgreifen und den Anschluß 61 kontaktieren. Würde hingegen die Batterie 60 in entgegengesetzter Richtung eingesetzt werden, so würde kein Kontakt zustande kommen, da der negative Anschluß 60b der Batterie 60 nicht durch die Öffnung 64 der Anlagefläche 63 hindurchgreifen kann. Die Beschädigung des Geräts durch ein falsches Einlegen der Batterien 60 in das Handstück 1 ist somit ausgeschlossen. Die Kontaktierung des negativen Anschlusses 60b der Batterie 60 erfolgt über einen weiteren Kontakt 62 im vorderen Bereich des Handstücks.

Wie eingangs erwähnt wurde, besteht ein wesentliches Merkmal des erfindungsgemäßen Handstücks 1 darin, dass dieses vollkommen unabhängig von externen Einrichtungen verwendet werden kann. Alle zur Durchführung der Untersuchung und Darstellung des Untersuchungsergebnisses erforderlichen Elemente sind innerhalb des Handstücks 1 angeordnet. Insbesondere erfolgt auch die Darstellung des Meßergebnisses, wie später noch näher erläutert wird, über das Handstück 1 selbst.

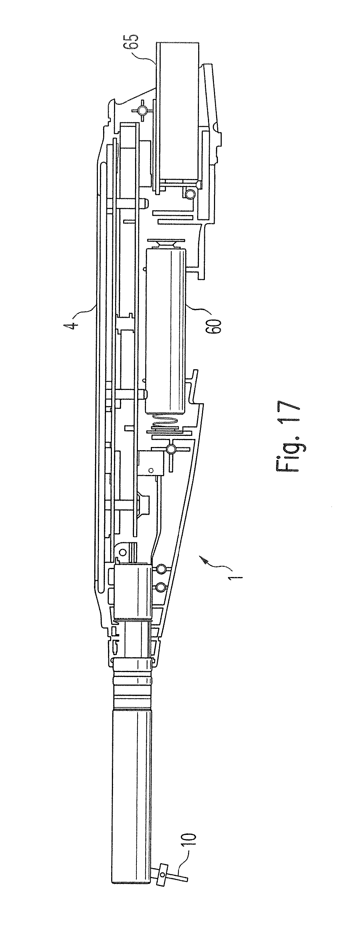

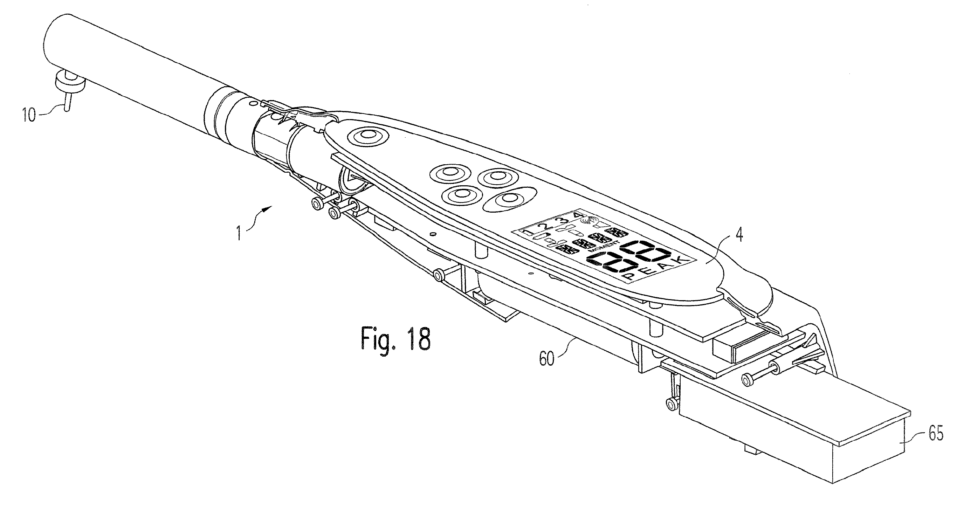

Es besteht zuweilen allerdings auch das Bedürfnis, das während einer Untersuchung erhaltene Meßergebnis an einen Zentralcomputer einer zahnärztlichen Praxis weiterzuleiten, in dem die Meßdaten unmittelbar gespeichert werden oder an dem eine zusätzliche Darstellung des Meßergebnisses erfolgt. Bei dem in den Fig. 17 und 18 dargestellten Handstück 1 ist demzufolge im hinteren Bereich des Handstücks 1 ein Kommunikationsmodul 65 angeordnet, über das die Meßdaten drahtlos an eine externe Einheit übermittelt werden können. Das Modul 65 kann dabei insbesondere dazu vorgesehen sein, die Meßdaten als Funkdaten zu übermitteln, was den Vorteil mit sich bringt, dass eine hohe Übertragungsqualität sichergestellt ist und somit die Gefahr von Fehlübertragungen nahezu ausgeschlossen ist. Alternativ dazu bestünde allerdings auch die Möglichkeit, die Daten mittels Infrarot- oder Ultraschallsignalen zu übertragen. Beide Lösungen können sehr kostengünstig realisiert werden, besitzen allerdings im Vergleich zur Funkübertragung eine geringere Reichweite. Dies kann jedoch unter Umständen sogar erwünscht sein, wenn vermieden werden soll, dass Störungen zwischen zwei in benachbarten Räumen angeordneten Geräten auftreten.

Ein besonderer Vorteil der Übertragung der Daten mittels Ultraschall besteht darin, dass der an dem Handstück 1 vorgesehene Lautsprecher, der primär zur Darstellung der Meßergebnisse benutzt wird, gleichzeitig auch zur Datenübertragung verwendet werden kann. Dies bedeutet, dass auf ein zusätzliches Element zur Datenübertragung verzichtet werden kann und lediglich eine geeignete Ansteuerung des Lautsprechers sichergestellt werden muß.

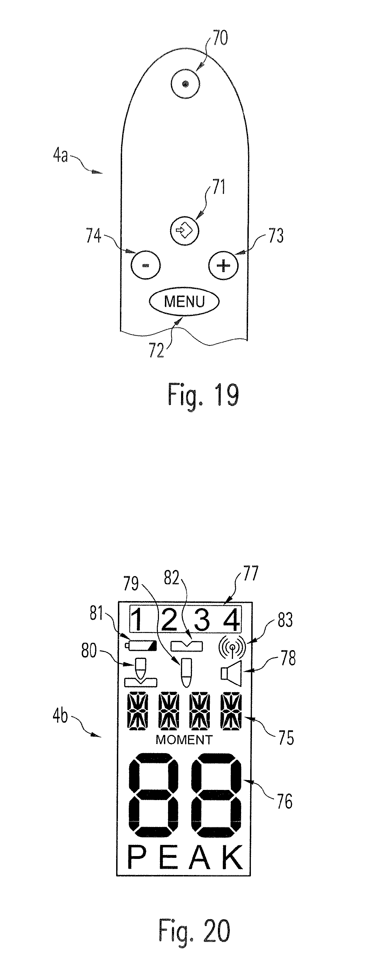

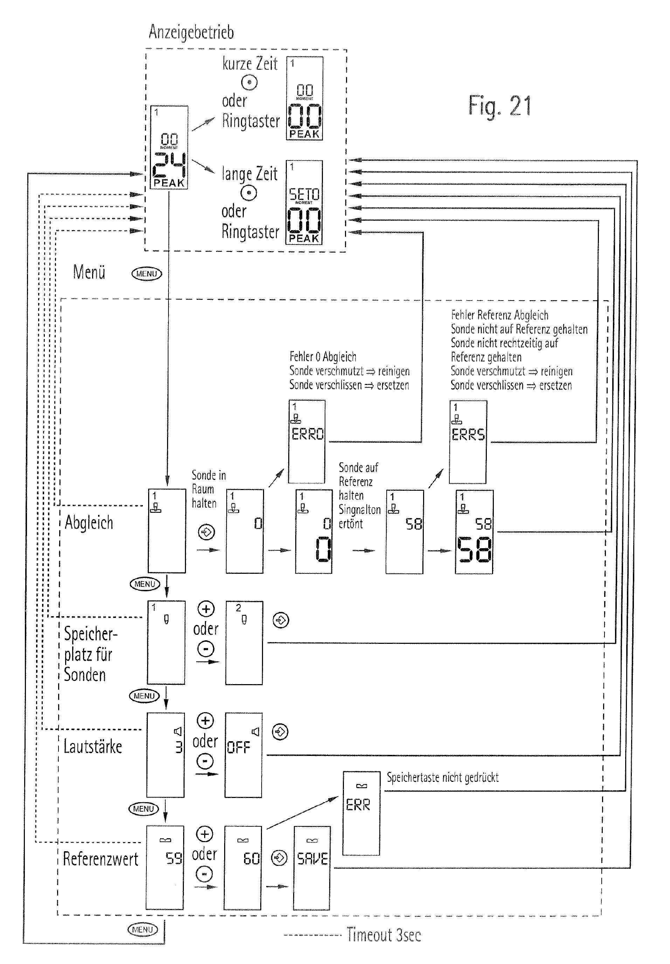

Anhand der Fig. 19 bis 21 sollen abschließend die verschiedenen Funktionen der erfindungsgemäßen Untersuchungseinrichtung sowie die Darstellung und Anzeige des Meßergebnisses näher beschrieben werden. Die Fig. 19 und 20 zeigen dabei das an der Oberseite des Handstücks befindliche Bedienfeld 4a zur Aktivierung bestimmter Funktionen sowie die Anzeigeeinheit 4b zur Darstellung der aktuellen Funktion bzw. des Meßergebnisses, während hingegen Fig. 21 das Konzept der Bedienerführung für das erfindungsgemäße Handstück 1 schematisch darstellt.

Das Bedienfeld 4a besteht aus insgesamt fünf Tasten, nämlich aus einer Zentraltaste 70, einer Speichertaste 71, einer Menütaste 72 sowie aus zwei Wähltasten 73 und 74. Die Funktion der einzelnen Tasten wird später anhand des Bedienschemas von Fig. 21 erläutert. Das Anzeigefeld 4b weist zum einen eine erste Digitalanzeige 75 zur Darstellung des momentanen Meßwertes, eine zweite Anzeige 76 zur Darstellung des Maximalwertes sowie eine Anzeige 77 zur Darstellung der Nummer der verwendeten Sonde dar. Ferner sind sechs Leuchtsymbole 78 bis 83 vorgesehen, durch welche der Status, in dem sich das Handstück 1 gerade befindet, oder ein Aktion, die gerade durchgeführt wird, angezeigt wird.

Mit Hilfe der Zentral- oder Multifunktionstaste 70 kann zum einen das Handstück vollständig ein- und eingeschaltet werden, darüber hinaus kann der Maximalwert zurückgesetzt bzw. eine Offsetkorrektur durchgeführt werden.

Die Funktion der Speichertaste 71 hingegen hängt davon ab, ob gerade der Menümodus aktiv ist oder nicht. Für den Fall, dass das Menü gerade nicht aktiviert wurde, wird durch das Drücken der Speichertaste 71 eine Übermittlung des aktuellen Peak-Werts über die drahtlose Verbindung zu einer zentralen Recheneinheit initiiert, was durch das Aufleuchten des Leuchtsymbols 83 verdeutlicht wird. Befindet sich hingegen das Handstück 1 gerade in einem bestimmten Menüpunkt, so wird durch das Drücken der Speichertaste 71 der aktuell eingestellte Wert abgespeichert und das Menü verlassen.

Auch die Funktion der beiden Wähltasten 73 und 74 hängt davon ab, ob das Menü gerade aktiv ist oder nicht. Bei inaktivem Menü kann über die drahtlose Verbindung ein Signal an eine zentrale Darstellungseinheit übermittelt werden, welches eine Vorwärtsbewegung in einem auf einem Display dargestellten Zahnschema veranlaßt. In gleicher Weise wird bei nicht aktivem Menü mit Hilfe der Taste 74 eine Rückwärtsbewegung in dem Zahnschema veranlaßt, so dass bei Tasten 73 und 74 in diesem Stadium als Navigationstasten verwendet werden. Bei aktivem Menü hingegen kann mit den Tasten 73 und 74 der aktuelle Zahlenwert des in dem entsprechenden Menü-Punkts einzustellenden Parameters erhöht bzw. erniedrigt werden.

Die Menütaste 72 schließlich dient zum Aufruf eines bestimmten Menüpunkts, wobei in rotierender Form zwischen den verschiedenen Punkten gewählt wird. Die verschiedenen Menüpunkte lauten *Abgleich, Sondenanwahl, Lautstärke, Referenzwert* und *Anzeigebetrieb,* wobei die verschiedenen Funktionen dieser Menüpunkte dem Schema in Fig. 21 entnommen werden können.

Der Menüpunkt *"Abgleich"* dient dazu, einen Abgleich der Untersuchungsvorrichtung durchzuführen. Nach einem Drücken der Speichertaste 71 wird hierzu zunächst ein Null-Signal aufgenommen, welches erhalten wird, indem die Meßsonde in den freien Raum gerichtet wird. Anschließend ist die Sonde auf einen Referenzkörper zu richten und es wird eine weitere Messung durchgeführt, wobei das hierbei erhaltene Meßergebnis zum Abgleichen der Untersuchungsvorrichtung herangezogen wird.

Der Menüpunkt *"Sondenanwahl"* kann dazu verwendet werden, der aktuell montierten Sonde eine bestimmte Nummer zuzuordnen. Dies ist bei einer späteren Auswertung des Meßergebnisses hilfreich, da jedem Meßergebnis eine bestimmte Sonde zugeordnet ist.

Der dritte Menüpunkt *"Lautstärke"* dient dazu, die Lautstärke des über den Lautsprecher abgegebenen Meßsignals einzustellen. Mit Hilfe des Menüpunkts *"Referenzwert"* schließlich kann ein Referenzwert bestimmt werden, der angibt, ab welcher Intensität des Meßsignals auf das Vorhandensein von Karies geschlossen wird bzw. eine akustische Anzeige des Messergebnisses erfolgt.

Im Normalbetriebszustand befindet sich die erfindungsgemäße Vorrichtung im sogenannten Anzeigebetrieb. Hierbei wird in der Momentanwert-Anzeige 75 bei einer Aktivierung der Lichtquelle für die Anregungsstrahlung der momentane Meßwert dargestellt, der über die Intensität der erfaßten Antwortstrahlung Auskunft gibt. Gleichzeitig wird in der Spitzenwert-Anzeige 76 der Peak-Wert der aktuellen Messung dargestellt, der durch einen permanenten Vergleich mit den aktuellen Meßwerten ermittelt wird. Die Speicherung dieses Peak-Werts ist deshalb von Vorteil, da hierdurch zu einem späteren Zeitpunkt der Messung auf einfache Weise diejenige Stelle wieder gefunden werden kann, an der die größte Ansammlung einer fluoreszierende Substanz diagnostiziert wurde.

In dem Anzeigebetrieb besteht ferner die Möglichkeit, durch ein langes Drücken der Multifunktionstaste 71 eine Offsetkorrektur durchzuführen, also den aktuellen Nullwert neu festzulegen. Durch ein kurzzeitiges Drücken der Multifunktionstaste hingegen wird der Peak-Wert auf den Wert Null zurückgesetzt und damit eine neue Messung gestartet.

Das erfindungsgemäße Benutzerschema ist besonders eingängig und verständlich, da es sich um ein einheitliches Bedienschema für jeden einzelnen Menüpunkt handelt. Die Bedienung der erfindungsgemäßen Untersuchungsvorrichtung kann somit bereits nach kurzer Zeit auch von nicht erfahrenen Personen durchgeführt werden.

Insgesamt wird somit durch die vorliegende Erfindung ein Untersuchungsgerät zur Untersuchung von Zahnoberflächen angegeben, welches sich durch seine besonders einfache Handhabung auszeichnet, gleichzeitig allerdings auch den Anforderungen an eine ausreichende Hygiene gerecht wird.

## Patentansprüche

1. Zahnärztliche Vorrichtung zum Untersuchen der optischen Eigenschaften von Zahngewebe, insbesondere zum Erkennen von Karies, Plaque, bakteriellem Befall, Konkrementen und Zahnstein, mit
Mitteln zum Erzeugen einer Anregungsstrahlung (A), welche auf einen zu untersuchenden Zahngewebebereich zu lenken ist,
Erfassungsmitteln (40) und Auswertemitteln zum Erfassen und Bewerten einer von dem bestrahlten Zahngewebebereich als Antwort auf die Bestrahlung entstehenden Antwortstrahlung (F), sowie
Anzeigemitteln (4b) zum Anzeigen eines von den Auswertemitteln auf Basis der erfassten Antwortstrahlung ermittelten Messergebnisses,
wobei die Mittel zum Erzeugen der Anregungsstrahlung (A) und die Erfassungsmittel (40) in ein zahnärztliches Handstück (1) integriert sind,
**dadurch gekennzeichnet,**
**dass** mindestens auch die Anzeigemittel (4b) in oder an dem Handstück (1) angeordnet sind,
wobei die Erfassungsmittel (40) eine in dem Handstück (1) angeordnete Fotodiode (42) zum Erfassen der Antwortstrahlung (F) umfassen.

2. Vorrichtung nach Anspruch 1,
**dadurch gekennzeichnet,**
**dass** die Fotodiode (42) an einem Ende eines Lichtleiters (41) zum Übermitteln der Antwortstrahlung (F) angeordnet ist.

3. Vorrichtung nach Anspruch 2,
**dadurch gekennzeichnet,**
**dass** die Fotodiode (42) in einem Gehäuse (43) angeordnet ist, welches eine Öffnung (45) aufweist, in die der Lichtleiter (41) mündet.

4. Vorrichtung nach Anspruch 3,
**dadurch gekennzeichnet,**
**dass** das Gehäuse (43) einen Schnappverschluss zum Anschließen des Lichtleiters (41) aufweist.

5. Vorrichtung nach Anspruch 3 oder 4,
**dadurch gekennzeichnet,**
**dass** in das Gehäuse (43) ein zwischen dem Lichtleiterende und der Fotodiode (42) angeordnetes Filter (46) integriert ist.

6. Zahnärztliche Vorrichtung zum Untersuchen der optischen Eigenschaften von Zahngewebe, insbesondere zum Erkennen von Karies, Plaque, bakteriellem Befall, Konkrementen und Zahnstein, mit
Mitteln zum Erzeugen einer Anregungsstrahlung (A), welche auf einen zu untersuchenden Zahngewebebereich zu lenken ist,
Erfassungsmitteln (40) und Auswertemitteln zum Erfassen und Bewerten einer von dem bestrahlten Zahngewebebereich als Antwort auf die Bestrahlung entstehenden Antwortstrahlung (F), sowie
Anzeigemitteln (4b) zum Anzeigen eines von den Auswertemitteln auf Basis der erfassten Antwortstrahlung ermittelten Messergebnisses,
wobei die Mittel zum Erzeugen der Anregungsstrahlung (A) und die Erfassungsmittel (40) in ein zahnärztliches Handstück (1) integriert sind,
und wobei die Erfassungsmittel (40) eine in dem Handstück (1) angeordnete Fotodiode (42) zum Erfassen der Antwortstrahlung (F) umfassen, welche an einem Ende eines Lichtleiters (41) zum Übermitteln der Antwortstrahlung (F) angeordnet ist,
**dadurch gekennzeichnet,**
**dass** die Fotodiode (42) in einem Gehäuse (43) angeordnet ist, welches eine Öffnung (45) aufweist, in die der Lichtleiter (41) mündet.
